(19) Europäisches Patentamt
European Patent Office
Office européen des brevets

(11) Veröffentlichungsnummer: **0 041 171**
**B1**

(12) # EUROPÄISCHE PATENTSCHRIFT

(45) Veröffentlichungstag der Patentschrift: **29.06.83**

(51) Int. Cl.³: **C 07 C 119/048,** C 07 C 118/02, C 07 C 103/375, C 07 C 102/00

(21) Anmeldenummer: **81103789.4**

(22) Anmeldetag: **18.05.81**

(54) **2-Chlor-5-nitro-phenylisocyanat, Verfahren zu seiner Herstellung und seine Verwendung.**

(30) Priorität: **29.05.80 DE 3020444**

(43) Veröffentlichungstag der Anmeldung: **09.12.81 Patentblatt 81/49**

(45) Bekanntmachung des Hinweises auf die Patenterteilung: **29.06.83 Patentblatt 83/26**

(84) Benannte Vertragsstaaten: **DE FR GB**

(56) Entgegenhaltungen:
**DE-A-1 618 770**
**DE-A-2 740 408**
**DE-B-1 593 049**

(73) Patentinhaber: **BAYER AG, Zentralbereich Patente, Marken und Lizenzen, D-5090 Leverkusen 1, Bayerwerk (DE)**

(72) Erfinder: **Blank, Heinz Ulrich, Dr., Am Geusfelde 35, D-5068 Odenthal (DE)**
Erfinder: **Wolters, Erich, Dr., Streffenweg 22, D-5162 Niederzier (DE)**
Erfinder: **Müller, Karl Walter, Dr., Bertha von Suttnerstrasse 29, D-5090 Leverkusen 1 (DE)**

**EP 0 041 171 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# 2-Chlor-5-nitro-phenylisocyanat, Verfahren zu seiner Herstellung und seine Verwendung

Die Erfindung betrifft die neue Verbindung 2-Chlor-5-nitro-phenylisocyanat, ein Verfahren zu seiner Herstellung und seine Verwendung zur Herstellung von substituierten Acetoacetaniliden.

Es wurde die Verbindung 2-Chlor-5-nitro-phenylisocyanat gefunden.

Weiterhin wurde ein Verfahren zur Herstellung von 2-Chlor-5-nitro-phenylisocyanat gefunden, das dadurch gekennzeichnet ist, dass man 2-Chlor-5-nitro-anilin oder dessen Hydrochlorid in einem inerten Lösungsmittel bei einer Temperatur von -10°C bis 200°C mit überschüssigem Phosgen umsetzt.

2-Chlor-5-nitro-anilin ist aus Berichte der Deutschen Chem. Gesellschaft 33, 3062 (1900) bekannt.

Die Umsetzung und weitere Behandlung mit überschüssigem Phosgen wird so durchgeführt, dass über die zur Umsetzung von einem Äquivalent Aminogruppe benötigte Menge von 1 Mol Phosgen hinaus ein Überschuss von beispielsweise 0,1 bis 10 Mol, bevorzugt 1 bis 4 Mol, besonders bevorzugt 2 bis 3 Mol Phosgen angewendet wird.

Im erfindungsgemässen Verfahren wird die Umsetzung mit Phosgen bei einer Temperatur von -10°C bis +200°C durchgeführt. Dabei kann es vorteilhaft sein, zunächst im unteren Temperaturbereich zu arbeiten und mit fortschreitender Reaktion auf eine höhere Temperatur überzugehen.

Die Umsetzung wird vorzugsweise zunächst bei einer Temperatur von -10°C bis +20°C, besonders bevorzugt bei 0 bis +5°C durchgeführt, wobei die Aminogruppe zum Teil in das Carbamidsäurechlorid und zum Teil in das Hydrochlorid umgewandelt wird. Die in dieser ersten Phase erhaltene Suspension wird sodann bei einer Temperatur von 30 bis 170°C, bevorzugt bei 50 bis 60°C weiter umgesetzt, bis die HCl-Entwicklung beendet ist und im allgemeinen eine klare Lösung vorliegt.

Für das erfindungsgemässe Verfahren sind Lösungsmittel geeignet, die unter den Reaktionsbedingungen mit den Reaktionspartnern nicht reagieren. Beispielsweise seien Kohlenwasserstoffe und chlorierte oder bromierte Kohlenwasserstoffe genannt. Bevorzugt werden aromatische Kohlenwasserstoffe, wie Benzol, Toluol oder Xylol, und chlorierte aromatische Kohlenwasserstoffe, wie Chlorbenzol, Dichlorbenzol oder Trichlorbenzol, eingesetzt. Besonders bevorzugt ist der Einsatz von Chlorbenzol oder o-Dichlorbenzol. Das Lösungsmittel wird in der 1- bis 10-fachen Gewichtsmenge, bevorzugt der 3- bis 5-fachen Gewichtsmenge, bezogen auf das eingesetzte 2-Chlor-5-nitro-anilin, angewendet.

Das erfindungsgemässe Verfahren kann durch die folgende summarische Gleichung wiedergegeben werden:

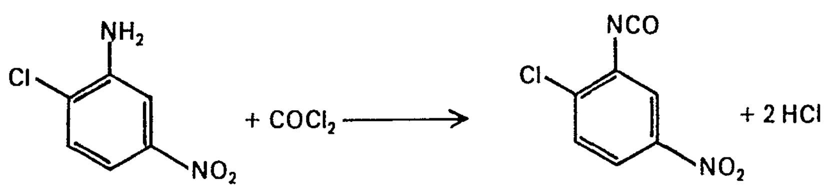

Zur Durchführung des erfindungsgemässen Verfahrens kann beispielsweise das Phosgen unter Kühlung in das gewählte Lösungsmittel eingeleitet werden und sodann das 2-Chlor-5-nitro-anilin als Feststoff eingetragen werden. Hierbei bildet sich zunächst ein Gemisch von Carbamidsäurechlorid und Hydrochlorid aus der ursprünglich vorhandenen Aminogruppe, welches als Suspension vorliegt. Sodann wird unter Rühren die Temperatur auf den weiter oben beschriebenen Bereich gebracht und weiteres Phosgen eingeleitet, bis die HCl-Entwicklung beendet ist und eine klare Lösung vorliegt. Hierbei soll ein dauernder Überschuss von Phosgen im Reaktionsgemisch vorliegen, was am fortdauernden Rückfluss des Phosgens im Kühler kontrolliert werden kann. Nach Beendigung der Reaktion kann das im Lösungsmittel gelöste überschüssige Phosgen, beispielsweise durch Destillation oder durch Ausblasen mit Stickstoff, zurückgewonnen werden. Zur Aufarbeitung und Isolierung der erfindungsgemässen Verbindung kann der grösste Teil des Lösungsmittels abdestilliert werden und das Produkt in der Kälte auskristallisiert werden. Es ist jedoch auch möglich, das Lösungsmittel völlig zu entfernen und den Rückstand durch Umkristallisation, beispielsweise aus Toluol, oder durch Destillation auf reines 2-Chlor-5-nitro-phenylisocyanat aufzuarbeiten. In den Fällen, in denen die Reinheit des Rohproduktes für die weitere Verarbeitung ausreichend ist, kann auf eine Isolierung des Produktes verzichtet werden und die erfindungsgemässe Verbindung in Form der als Reaktionslösung anfallenden etwa 20 bis 25 gew.-%igen Lösung des Isocyanats weiter verwendet werden. Hierbei wird, gegebenenfalls unter Anlegen eines schwachen Vakuums, und gegebenenfalls bei erhöhter Temperatur HCl und Phosgen aus der Lösung entfernt und vom Lösungsmittel gegebenenfalls so viel abdestilliert, dass die gewünschte Konzentration in der verbleibenden Lösung vorliegt.

Das erfindungsgemässe 2-Chlor-5-nitro-phenylisocyanat kann beispielsweise zur Herstellung von 2-Chlor-5-nitro-aniliden, bevorzugt zur Herstellung von substituierten Acetoacetaniliden der Formel

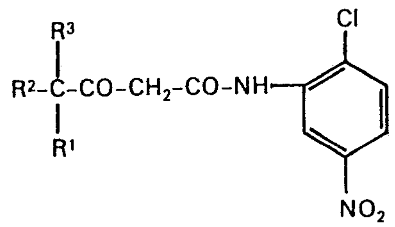
(I),

in der
$R^1$, $R^2$ und $R^3$ unabhängig voneinander $C_1$–$C_4$-Alkyl bedeuten,
verwendet werden.

Diese Verwendung ist durch die Reaktion eines Enamins mit dem Isocyanat und die darauf folgende Hydrolyse des Zwischenproduktes entsprechend der folgenden Formelgleichung gekennzeichnet:

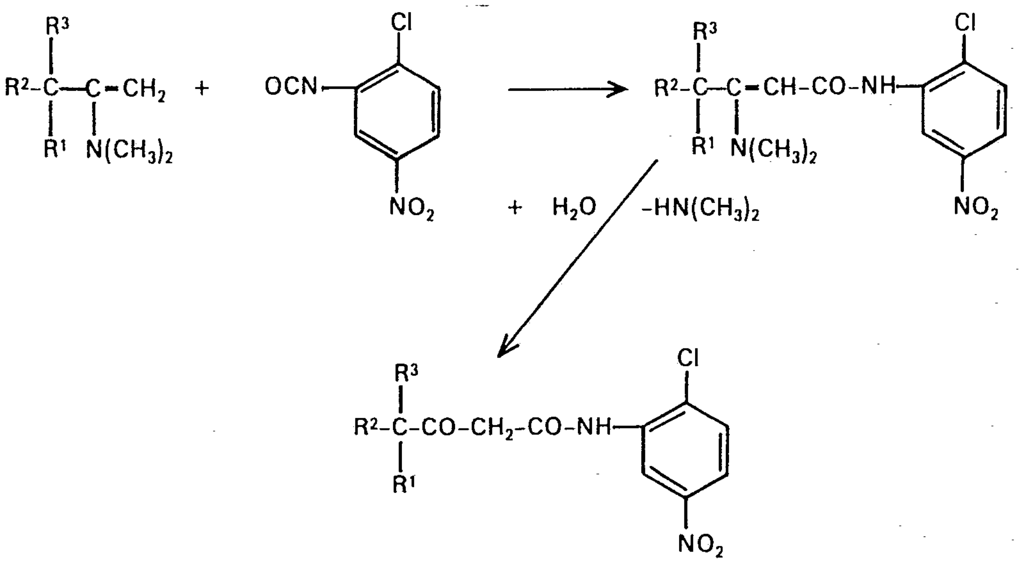


Die hierbei erhältlichen substituierten 2-Chlor-5-nitro-acetoacetanilide können durch katalytische Hydrierung mit Wasserstoff zu den entsprechenden 2-Chlor-5-amino-acetoacetaniliden umgesetzt werden, die ihrerseits mit 4-(2,4-di-tert.-amyl)-phenoxybuttersäurechlorid zu substituierten Acetoacet-2-chlor-5-[4-(2,4-di-tert.-amyl)-phenoxybutyramido]-aniliden umgesetzt werden können. Diese zuletzt genannten Anilide, beispielsweise das Pivaloylessigsäure-2-chlor-5-[4-(2,4-di-tert.-amyl)-phenoxybutyramido]-anilid der Formel

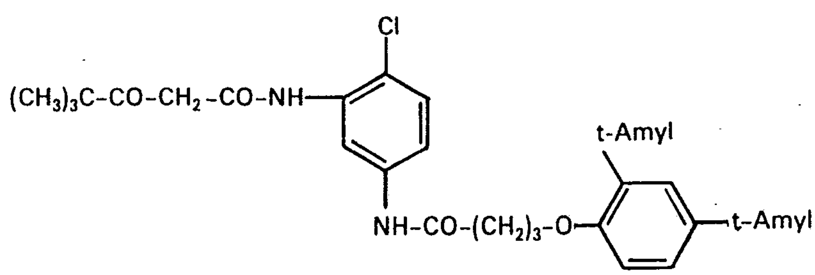
(II)

sind Zwischenprodukte für die Herstellung von Farbfilmkomponenten (US 3 265 506, US 3 384 657, DE-AS 11 24 356).

Beispiel 1

Zu einer Lösung von 145 ml (207 g, 2,1 Mol) Phosgen in 800 ml Chlorbenzol werden bei 0°C unter gutem Rühren im Laufe von ca. 30 Min. 180 g (1,0 Mol) 2-Chlor-5-nitro-anilin (96%ig) als Feststoff eingetragen. Die gelbbraune Suspension wird auf 55°C aufgeheizt. Dabei wird ein schwacher Strom Phosgen eingeleitet, so dass überschüssiges Phosgen am Rückfluss siedet. Unter HCl-Entwicklung klärt sich die Suspension im Laufe von 4 Stunden zur Lösung. Es wird noch eine Stunde nachphosgeniert.

Überschüssiges Phosgen und Chlorbenzol wird bis auf ein Restvolumen von 600 ml abdestilliert. Es entsteht eine etwa 23 gew.-%ige Lösung des 2-Chlor-5-nitro-phenylisocyanats in Chlorbenzol, die einen Phosgengehalt von weniger als 0,05% besitzt.

Gehalt nach Titration: 188,3 g Isocyanat ≙ 95% der Theorie

Bei der Destillation über eine beheizbare Kolonne werden bei einer Kopftemperatur von ca. 130°C und einem Druck von 1 mb 183 g 2-Chlor-5-nitro-phenylisocyanat (99%ig) erhalten. Dies entspricht einer Ausbeute von 93%. Der Schmelzpunkt des Produktes beträgt 93-95°C.

Beispiel 2

Zu 5,5 g 3,3-Dimethyl-2-dimethylamino-1-buten (93%ig; 0,04 Mol) werden unter Rühren im Verlauf von 20 Minuten 34,5 g einer 23,0%igen Rohlösung des 2-Chlor-5-nitrophenylisocyanates in Chlorbenzol (ca. 25 ml; 0,04 Mol) bei Raumtemperatur zugetropft. Es wird 5 Minuten nachgerührt. Dann werden 10 ml Wasser hinzugefügt und unter starkem Rühren etwa 3,9 ml conc. HCl so zugetropft, dass ein pH-Wert von etwa 5 eingehalten wird. Dies nimmt ca. 1,5-2 Stunden in Anspruch. Es wird noch ½ Stunde nachgerührt. Das ausgefallene Produkt wird abgesaugt, die organische Phase auf ca. 10 ml eingeengt und auf −15°C abgekühlt, wobei weiteres Produkt ausfällt. Nach dem Trocknen im Vakuum bei 50°C bleiben 10,6 g (91% d.Th.) Pivaloyl-2-chlor-5-nitroacetanilid mit einem Schmelzpunkt von 116-119°C.

**Patentansprüche**

1. 2-Chlor-5-nitro-phenylisocyanat.
2. Verfahren zur Herstellung von 2-Chlor-5-nitro-phenylisocyanat, dadurch gekennzeichnet, dass man 2-Chlor-5-nitro-anilin oder dessen Hydrochlorid in einem inerten Lösungsmittel bei einer Temperatur von -10°C bis +200°C mit überschüssigem Phosgen umsetzt.
3. Verfahren nach Anspruch 2, dadurch gekennzeichnet, dass man zunächst im unteren Temperaturbereich arbeitet und mit fortschreitender Reaktion auf eine höhere Temperatur übergeht.
4. Verfahren nach Anspruch 2 und 3, dadurch gekennzeichnet, dass man das 2-Chlor-5-nitroanilin oder dessen Hydrochlorid zunächst bei -10°C bis +20°C mit überschüssigem Phosgen umsetzt und das Reaktionsgemisch bei einer Temperatur von 30 bis 170°C mit weiterem Phosgen bis zur Beendigung der HCl-Entwicklung weiter behandelt.
5. Verfahren nach Anspruch 2 bis 4, dadurch gekennzeichnet, dass über die zur Umsetzung der Aminogruppe benötigte Menge hinaus ein Überschuss von 0,1 bis 10 Mol Phosgen angewendet wird.
6. Verfahren nach Anspruch 2 bis 5, dadurch gekennzeichnet, dass der Phosgenüberschuss 1 bis 4 Mol beträgt.
7. Verfahren nach Anspruch 2 bis 6, dadurch gekennzeichnet, dass in einem gegebenenfalls chlorierten aromatischen Kohlenwasserstoff als Lösungsmittel gearbeitet wird.
8. Verfahren nach Anspruch 2 bis 7, dadurch gekennzeichnet, dass in Chlorbenzol oder o-Dichlorbenzol als Lösungsmittel gearbeitet wird.
9. Verwendung von 2-Chlor-5-nitro-phenylisocyanat zur Herstellung von 2-Chlor-5-nitroaniliden.

**Claims**

1. 2-Chloro-5-nitro-phenyl isocyanate.
2. Process for the preparation of 2-chloro-5-nitro-phenyl isocyanate, characterised in that 2-chloro-5-nitro-aniline or the hydrochloride thereof is reacted in an inert solvent at a temperature of -10°C to +200°C with excess phosgene.
3. Process according to Claim 2, characterised in that the reaction is initially carried out in the lower temperature range and the temperature is changed to a higher temperature as the reaction proceeds.
4. Process according to Claim 2 and 3, characterised in that 2-chloro-5-nitro-aniline or the hydrochloride thereof is initially reacted at -10°C to +20°C with excess phosgene and the reaction mixture is further treated at a temperature of 30 to 170°C with further phosgene until the evolution of HCl has ceased.
5. Process according to Claim 2 to 4, characterised in that an excess of 0.1 to 10 mols of phosgene is used in addition to the amount required for conversion of the amino group.
6. Process according to Claim 2 to 5, characterised in that the phosgene excess is 1 to 4 mols.
7. Process according to Claim 2 to 6, characterised in that the reaction is carried out in an optionally chlorinated aromatic hydrocarbon as the solvent.
8. Process according to Claim 2 to 7, characterised in that the reaction is carried out in chlorobenzene or o-dichlorobenzene as the solvent.
9. Use of 2-chloro-5-nitro-phenyl isocyanate for the preparation of 2-chloro-5-nitro-anilides.

**Revendications**

1. Le 2-chloro-5-nitrophénylisocyanate.
2. Procédé de production du 2-chloro-5-nitrophénylisocyanate, caractérisé en ce qu'on fait réagir de la 2-chloro-5-nitro-aniline ou son chlorhydrate dans un solvant inerte à une température de -10 à +200°C avec du phosgène en excès.
3. Procédé suivant la revendication 2, caractérisé en ce qu'on opère tout d'abord dans la partie basse de la plage de températures et on passe à une température élevée à mesure que la réaction progresse.
4. Procédé suivant les revendications 2 et 3, caractérisé en ce qu'on fait réagir la 2-chloro-5-nitro-aniline ou son chlorhydrate d'abord entre -10 et +20°C avec du phosgène en excès et on

traite ensuite le mélange réactionnel à une température de 30à 170°C avec une quantité additionnelle de phosgène jusqu'à ce que le dégagement de HCl soit terminé.

5. Procédé suivant les revendications 2 à 4, caractérisé en ce qu'on utilise, en plus de la quantité nécessaire à la réaction du groupe amino, un excès de phosgène de 0,1 à 10 moles.

6. Procédé suivant les revendications 2 à 5, caractérisé en ce que l'excès de phosgène va de 1 à 4 moles.

7. Procédé suivant les revendications 2 à 6, caractérisé en ce qu'on opère dans un hydrocarbure aromatique éventuellement chloré utilisé comme solvant.

8. Procédé suivant les revendications 2 à 7, caractérisé en ce qu'on opère dans du chlorobenzène ou du o-dichlorobenzène utilisé comme solvant.

9. Utilisation du 2-chloro-5-nitro-phényl-isocyanate pour la production de 2-chloro-5-nitro-anilides.